# EUROPEAN PATENT SPECIFICATION

(11) **EP 3 894 853 B1**
(45) Date of publication and mention of the grant of the patent: **29.11.2023**
(21) Application number: 19823963.4
(22) Date of filing: 13.12.2019
(51) Int. Cl.: G01N 33/543

(54) **MULTIPLEXED ASSAY**
MULTIPLEXTEST
DOSAGE MULTIPLEXÉ

(30) Priority: 14.12.2018 GB 201820413
(43) Date of publication of application: 20.10.2021
(73) Proprietor: Osler Diagnostics Limited, Oxford OX1 1JD (GB)
(72) Inventor: BALDWIN, Dene, Oxford OX1 1JD (GB); PSARROS, Konstantinos, Oxford OX1 1JD (GB); EDINGTON, David Wiliam Neil, Oxford OX1 1JD (GB)
(74) Representative: Kilburn & Strode LLP
(86) International application number: PCT/GB2019/053549
(87) International publication number: WO 2020/120990

(56) References cited:
- WO-A1-2011/057347
- WO-A2-2004/071641
- US-A1- 2013 115 619

## Description

### BACKGROUND TO THE INVENTION

The present application relates to a tag/anti-tag system for multiplexed assays. In particular the present application relates to a universal multiplex platform device comprising an array of different anti-tags, and methods using the device or system to determine the presence or amount of multiple analytes in a sample.

Immunoassays are often used for the detection of a specific analyte within a sample. There are a number of different immunoassay formats. For example, the following are common formats:
- Two site, immunometric or sandwich assay
- Competitive immunoassay
- Competitive immunoassay (inverted)
- Direct immunoassay for detecting patient antibodies
- Indirect immunoassay for detecting patient antibodies

These standard immunoassays all rely on the separation of antibody bound analyte and free (non-bound) antibody. They rely on the immobilisation of either an antibody or antigen to a surface and can be used to measure antigens or antibodies.

A multiplex assay is a type of assay used to simultaneously measure multiple analytes in a single run/cycle or assay device. The conventional multiplex approach requires the deposition of individual binding reagents (antibodies or antigens) in a fixed array to make a test panel. This requires complex equipment & processes to deliver either on continuous reel systems or discreet dispensing into a device array. Increasing the number of analytes in a panel increases the risk of batch failure since only one assay has to fall out of specification for the entire panel to fail.

Therefore, there is a requirement and a need in the technical field to develop a robust and sensitive multiplex immunoassay method and device that can be used to accurately detect and/or quantify a wide range of target analytes.

It is against this background that the present invention has arisen.

The present inventors have developed multiplex devices and multiplex assay methods that use multiple tag/anti-tag pairs.

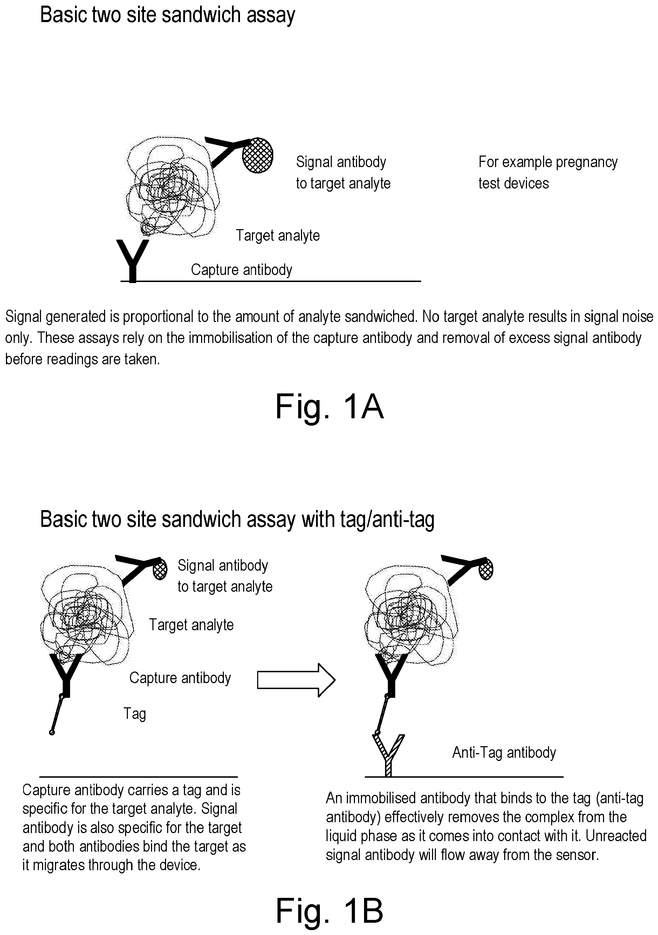

The use of single tag/anti-tag systems (e.g. biotin-avidin) to facilitate the immobilisation of single analyte chemistry, is known in the prior art. These rely on the separation of tagged and non-tagged complexes using immobilised anti-tags. Figures 1-5 show each standard immunoassay listed above alongside a version using a tag/anti-tag system.

Epitope tags are used extensively in proteomics & protein chemistry and can be an amino acid sequence or molecule attached to or assembled into a target analyte (protein or nucleic acid). The matching binding reagent - the anti-tag is typically an antibody but could also be an aptamer, affimer, molecularly imprinted polymer (MIP) or other binding reagent (e.g. antibody fragment, nucleic acids (oligonucleotides), peptides (e.g. flag tags), enzymes and chemicals (e.g. FITC)). The most common tag/anti-tag system is biotin-avidin but there are many options available.

Abbott US5952173 relates to devices & methods utilising array of structures for analyte capture and is focused on a single analyte detection approach. It discloses use of an immobilised reagent capable of binding an ancillary specific binding member. It mentions multiple capture sites. It also mentions multiple analyte detection, but in this context the example described is a drugs of abuse assay which comprises immobilised antibodies specific for the different analytes. It does not disclose an array of different anti-tags immobilised on a solid surface for immobilising tagged immune complexes that form in the liquid phase.

### SUMMARY OF THE INVENTION

The device and methods provided herein utilise a tag/anti-tag system to detect multiple target analytes in multiplexed assays, wherein an array of different anti-tags is immobilised on a solid surface.

In an aspect of the present invention, there is provided a microfluidic device for conducting multiplex immunoassays to detect multiple target analytes in a liquid sample using a set of tags and anti-tags, wherein the device comprises an array of different anti-tags immobilised on a surface of a sensor for measuring a signal, wherein each anti-tag is arranged to immobilise a tagged antibody or tagged antigen thereby in use separating tagged immune complexes from the liquid phase affecting the signal measured; wherein the device comprises a sensor chamber for tagged immune complexes to form in the liquid phase, and the device comprises one or more detection zones comprising the array of different anti-tags for immobilising tagged immune complexes; wherein the device comprises a microfluidic delay channel between the sensor chamber and the detection zone(s) to increase the liquid phase incubation time of reagents that react to form tagged immune complexes before reaching the anti-tags; and wherein in use liquid comprising the tagged immune complexes flows or is fluidically driven from the sensor chamber to the detection zone(s).

Thus, in another aspect of the disclosure which is not claimed, provided herein is a device for conducting multiplex immunoassays to detect multiple target analytes in a liquid sample using a set of tags and anti-tags, wherein the device comprises an array of different anti-tags immobilised on a surface of a sensor for measuring a signal, wherein each anti-tag is arranged to immobilise a tagged antibody or tagged antigen thereby in use separating tagged immune complexes from the liquid phase affecting the signal measured.

In another aspect of the disclosure which is not claimed, there is provided a microfluidic device for conducting multiplex immunoassays to detect multiple target analytes in a liquid sample using a set of tags and anti-tags, wherein the device comprises an array of different anti-tags immobilised on a surface of a sensor for measuring a signal, wherein each anti-tag is arranged to immobilise a tagged antibody or tagged antigen thereby in use separating tagged immune complexes from the liquid phase affecting the signal measured; wherein the device comprises a sensor chamber for tagged immune complexes to form in the liquid phase, and the device comprises one or more detection zones comprising the array of different anti-tags for immobilising tagged immune complexes; and wherein in use liquid comprising the tagged immune complexes flows or is fluidically driven from the sensor chamber to the detection zone(s).

Advantageously, this tag/anti-tag format, allows a single universal platform device to be manufactured and utilised for many different multiple analyte tests e.g. a cardiac panel, an infectious disease panel, a drugs of abuse panel, amongst many others.

Advantageously, this tag/anti-tag format allows the primary incubation between target specific antibodies and antigens to happen in the liquid phase. The interactions in a liquid state are faster than liquid-solid state (e.g. in the case of immobilised capture antibodies). Thus, faster binding kinetics allows for faster detection, more sensitivity and better performance. Incubation time for the primary incubation can be easily varied e.g. lengthened to allow for detection of low concentration analytes. The multiplex tag/anti-tag format allows for a more automated approach to multiplexed assays.

Advantageously, this tag/anti-tag format allows different immunoassay formats to be simultaneously run on the same panel.

Suitably, the device comprises a sensor chamber for tagged immune complexes to form in the liquid phase, and the device comprises one or more detection zones comprising the different anti-tags for immobilising the tagged immune complexes. Liquid can pass passively or actively from the sensor chamber to the detection zone(s). The device may be a microfluidic device wherein liquid (e.g. tagged immune complexes) flows or is fluidically driven from the sensor chamber to the detection zone(s). Typically the sensor chamber, for incubation of reagents that react to form tagged immune complexes, is upstream of the detection zone(s). The device comprises a microfluidic delay channel between the sensor chamber and the detection zone(s) to increase the liquid phase incubation time of reagents that react to form tagged immune complexes before reaching the anti-tags. For example, the hydraulic resistance of the microfluidic delay channel can be adjusted to provide the intended delay by selecting the appropriate cross-sectional area and/or length of the channel.

In some embodiments, the detection/signal measurement comprises colorimetric detection, fluorometric detection, faradaic or non-faradaic impedance spectroscopy, or redox active precipitate detection.

In some embodiments, the device comprises an array of electrodes with a different anti-tag on each electrode, optionally wherein each anti-tag is adsorbed or chemically linked to the electrode, and optionally wherein the electrode is a gold electrode.

In some embodiments, either i) a portion of the surface of the sensor comprises a conductive or semi conductive polymer or ii) the surface of the sensor comprises the surface of one or more electrodes.

The anti-tag antibodies or molecules can be tethered on the surface of the electrode in a similar way as a conventional antibody. This can include passive adsorption, coupling chemistries (EDC/NHS, epoxy, maleimide, etc.). The tags can be added to an antibody by wither chemical coupling (EDC/NHS, epoxy, maleimide, iodoacetamide, tosyl, etc) or in some instances can be incorporated into the structure of the antibody during production using recombinant technologies (addition of flag/6-his and cys tags).

In some embodiments, the multiple target analytes comprise antibodies and/or antigens.

The anti-tag may be a group or a species. In some embodiments, the anti-tag is an antibody, aptamer, affimer, molecularly imprinted polymer (MIP) or other binding reagent (e.g. fragments, nucleic acids. Other examples of suitable anti-tags: Nucleic acids (oligonucleotides), peptides (e.g. flag tags), enzymes and chemicals (e.g. FITC)). In some embodiments, wherein the anti-tag is an antibody, the anti-tag antibody/tag pairs may be any of the pairs as set out in Table 1 herein e.g. the anti-tag antibody is avidin/anti-biotin and the corresponding tag is biotin or anti-flag^{®} tag antibody against flag^{®} tag, or anti-His-6 antibody against His-6, or anti-c-myc antibody against c-myc, or anti-biotin acceptor peptide antibody against biotin acceptor peptide, or anti- V5 antibody against V5 tag or anti- glutathione S-transferase (GST) antibody against glutathione S-transferase tag, or the anti-tag antibody is sheep anti-mouse ab and the corresponding tag is primary ab e.g. mouse, or anti-FITC against FITC, or anti-HRP against HRP, or anti-MPB against R Maltose binding protein; or anti-streptavidin/biotin against streptavidin.

In some embodiments, the device comprises an array of at least: 3 or 4 or 5 or 6 or 7 or 8 or 9 or 10 or 11 or 12 or 13 or 14 or 15 different anti-tags. In some embodiments, the device comprises an array of 6 different anti-tags, optionally the 6 different anti-tags are antibodies against: FITC or biotin, Flag, His-6, c-Myc, V5 and GST.

The tag/anti-tag format enables a universal platform device as described herein and can be used for infectious disease testing or cardiac testing or drug testing or immunostatus/allergy testing.

In some embodiments provided herein, the multiplex assay comprises one or more of the following immunoassay formats:
a. Two site immunometric or sandwich assay with anti-tag separation
b. Competitive immunoassay with anti-tag separation
c. Competitive immunoassay (inverted) with anti-tag separation
d. Direct immunoassay for detecting antibodies with anti-tag separation
e. Indirect immunoassay for detecting antibodies with anti-tag separation.

In some embodiments, the array of different anti-tags is arranged to conduct immunoassays of at least two different formats on a single device. Thus, advantageously, different immunoassay formats can be simultaneously run on the same anti-tag array. For example, the array of anti-tags can be arranged to detect low levels of cortisol whilst also, at the same time, quantitating higher levels of immunoglobulin G and immunoglobulin A, and optionally also detecting testosterone and/or immunoglobulin M. Suitably, the cortisol/testosterone would be detected using a competitive immunoassay with anti-tag separation and for the immunoglobulins the assay format would be sandwich. This type of array of anti-tags would be useful for example for sports medicine testing. The detection of cortisol and immunoglobulins could equally be applied to a Wellness Test for office workers looking at stress and immune function. In an alternative embodiment with different immunoassay formats on the same device, the device could be for the detection of at least one anticancer drug as well as various tumour markers. Suitably, the assay format would be competitive for the anticancer drug(s) and sandwich for the markers.

In some embodiments, the array of anti-tags may be arranged to detect low levels of cortisol whilst also quantitating higher levels of immunoglobulin G and immunoglobulin A, and optionally also detecting testosterone and/or immunoglobulin M; wherein the device comprises a competitive immunoassay format for detecting cortisol and optionally testosterone, and a sandwich assay format for detecting immunoglobulins G and A and optionally immunoglobulin M.

In some embodiments, the device may comprise an anti-tag panel for the detection of at least one anti-cancer drug as well as various tumour markers.

In a further aspect that is not claimed, provided herein is a method for detecting multiple target analytes in a liquid sample using a device provided herein. In embodiments, the method is for infectious disease testing, cardiac testing or drug testing or immunostatus/allergy testing.

In another aspect that is not claimed, there is provided a method for detecting multiple target analytes in a liquid sample, using a device according to any of any of the aspects of the invention, wherein at least one assay is a two site immunometric sandwich assay with tag/anti-tag for detecting target analyte X, wherein a capture antibody carries the tag and is specific for the target analyte X, and wherein a signal antibody is also specific for the target analyte X, and wherein both antibodies bind the target analyte X in the liquid phase as it migrates through the device, and wherein an immobilised anti-tag binds to the tag and thereby removes the complex from the liquid phase as it comes into contact with it, and wherein unreacted signal antibody flows away from the sensor.

In some embodiments, the method comprises at least one assay which is a two site immunometric sandwich assay with tag/anti-tag for detecting target analyte X, wherein a capture antibody carries the tag and is specific for the target analyte X, and wherein a signal antibody is also specific for the target analyte X, and wherein both antibodies bind the target analyte X in the liquid phase as it migrates through the device, and wherein an immobilised anti-tag binds to the tag and thereby removes the complex from the liquid phase as it comes into contact with it, and wherein unreacted signal antibody flows away from the sensor. The term "analyte X" used herein means one of the multiple target analytes to be detected.

In some embodiments, the method comprises at least one assay which is a competitive immunoassay with anti-tag separation for detecting target analyte X, wherein target analyte X and analyte labelled with a tag compete for binding on the antibody which carries a signal, wherein on reaching the anti-tag, tagged analyte X becomes immobilised, such that in the absence of target analyte the signal will be maximal, whereas high target analyte concentrations will yield minimal signal.

In some embodiments, the method comprises at least one assay which is a competitive inverted immunoassay with anti-tag separation for detecting target analyte X, wherein target analyte X and analyte labelled with signal compete for binding on an antibody that carries a tag, wherein on reaching the anti-tag the tagged antibody becomes immobilised, such that in the absence of target analyte the signal will be maximal and high target analyte concentrations will yield minimal signal.

In some embodiments, the method comprises at least one assay which is a direct immunoassay with anti-tag separation for detecting target analyte X wherein target analyte X is an antibody, wherein in the liquid phase the target analyte X binds a signal labelled antigen and the target analyte X is captured by a capture antibody with a tag, wherein the complex is separated when the immobilised anti-tag binds the tag on the capture antibody, and excess signal labelled antigen migrates away from the sensor. For example, the method can be for infectious disease testing wherein the device comprises 6 different anti-tags and wherein each tag labels a different infectious disease antigen (e.g. for detecting human IgG antibodies to Rubella, measles, mumps, HSV1, HSV2, CMV, Toxoplasma).

In some embodiments, for infectious disease testing, wherein the device may comprise 6 different anti-tags and wherein each tag labels a different infectious disease antigen.

In some embodiments, the method comprises at least one assay which is an indirect immunoassay with anti-tag separation for detecting target analyte X wherein target analyte X is an antibody, wherein in the liquid phase signal antibody binds the target analyte X which binds tagged antigen, and wherein tagged antigen becomes immobilised when bound to the anti-tag, and wherein the signal generated is proportional to the amount of target analyte X in the sample, and no target analyte X results in no signal, wherein excess unbound signal antibody is removed before signal readings are taken.

In some embodiments, the method according to any one of the aspects of the invention for cardiac testing or drug testing or immunostatus/allergy testing

In some embodiments according to any one of the aspects of the invention for detecting low concentration analytes wherein the device may comprise a microfluidic delay channel to increase the liquid phase incubation time of reagents that react to form a tagged immune complex before they reach the anti-tag.

In some embodiments, the complimentary anti-tags/tags may have medium to high binding affinity to allow for relatively short incubation times and rapid immobilisation of tagged immune complex. In some embodiments, the method is for detecting low concentration analytes and the device comprises a microfluidic delay channel to increase the liquid phase incubation time of reagents that react to form a tagged immune complex before they reach the anti-tag.

In some embodiments, the method comprises anti-tags/tag pairs that have medium to high binding affinity to allow for relatively short incubation times and rapid immobilisation of the tagged immune complex.

In some embodiments, the method comprises one or more of the following immunoassay formats:
a. Two site immunometric or sandwich assay with anti-tag separation
b. Competitive immunoassay with anti-tag separation
c. Competitive immunoassay (inverted) with anti-tag separation
d. Direct immunoassay for detecting antibodies with anti-tag separation
e. Indirect immunoassay for detecting antibodies with anti-tag separation;
and the detection/signal measurement comprises colorimetric detection, fluorometric detection, faradaic or non-faradaic impedance spectroscopy, or redox active precipitate detection.

In a another aspect which is not claimed, provided herein is a kit comprising a device as described herein together with reagents, wherein the reagents comprise the tag reagents, and optionally the signal reagents.

In a yet further aspect that is not claimed, provided herein is a method for detecting multiple target analytes in a test liquid sample using a set of tags and anti-tags, the method comprising the steps of:
a. providing a device comprising an array of different anti-tags immobilised on a surface of a sensor for measuring a signal, wherein each anti-tag is arranged to immobilise a tagged antibody or tagged antigen;
b. adding a test liquid sample to the device in the presence of reagents, comprising tag reagents, so that a tagged immune complex can form in the liquid phase;
thereby, as a result of binding between the anti-tags and tags, tagged immune complexes are separated from the liquid phase, affecting the signal measured by the sensor, and determining the presence or amount of each target analyte.

In another aspect which is not claimed, there is provided method for detecting multiple target analytes in a test liquid sample using a set of tags and anti-tags, the method comprising the steps of:
a. providing a microfluidic device comprising an array of different anti-tags immobilised on a surface of a sensor for measuring a signal, wherein each anti-tag is arranged to immobilise a tagged antibody or tagged antigen; and
b. adding a test liquid sample to the sensor chamber in the presence of reagents, comprising tag reagents, and optionally signal reagents e.g. signal antibody(s) or signal analyte(s)/antigen(s), so that a tagged immune complex can form in the liquid phase;
   wherein the array of different anti-tags are immobilised in a detection zone, and the tagged immune complex forms in the sensor chamber upstream of the detection zone and flows or is fluidically driven to the detection zone; thereby, as a result of the anti-tags and tags binding, tagged immune complexes are separated from the liquid phase, affecting the signal measured by the sensor, and determining the presence or amount of each target analyte.

In some embodiments, the reagents comprise signal reagents e.g. signal antibody(s) or signal analyte(s)/antigen(s).

In some embodiments, each anti-tag may be an antibody, aptamer, affimer or molecularly imprinted polymer (MIP).

In some embodiments when an anti-tag is an antibody, the anti-tag antibody may be avidin/anti-biotin and the corresponding tag may be biotin or anti-flag^{®} tag antibody against flag^{®} tag, or anti-His-6 antibody against His-6, or anti-c-myc antibody against c-myc, or anti- biotin acceptor peptide antibody against biotin acceptor peptide, or anti- V5 antibody against V5 tag or anti- glutathione S-transferase (GST) antibody against glutathione S-transferase tag, or the anti-tag antibody is sheep anti-mouse ab and the corresponding tag is primary ab e.g. mouse, or anti-FITC against FITC, or anti-HRP against HRP, or anti-MPB against R Maltose binding protein; or anti-streptavidin/biotin against streptavidin.

In some embodiments, the detection/signal measurement comprises colorimetric detection, fluorometric detection, faradaic or non-faradaic impedance spectroscopy, or redox active precipitate detection.

In some embodiments, either i) a portion of the surface of the sensor comprises a conductive or semi conductive polymer or ii) the surface of the sensor comprises the surface of one or more electrodes.

In some embodiments, the device is a microfluidic device, and the array of different anti-tags are immobilised in a detection zone, and the tagged immune complex forms in a sensor chamber upstream of the detection zone and flows or is fluidically driven to the detection zone.

In some embodiments, the device comprises multiple electrodes and there is different anti-tag on each electrode, and optionally wherein the detection/signal measurement comprises faradaic or non-faradaic impedance spectroscopy.

In some embodiments, after sufficient incubation time for the tag and anti-tag binding, a wash step is introduced to remove any unbound excess material (e.g. antibody or antigens).

The addition of tags to antibodies is well known in the art, and used in lab-based assays. However, in microfluidic systems, deposition strategies become increasingly important as many analytes need to be captured in the same sensor area, where space is very limited. If using traditional coating methods, addition of several different antibodies will be subject to different protocols for each antibody (if using passive adsorption). This can make the manufacturing process very difficult. Furthermore, if new analytes are to be introduced into the cartridge, a new experimental protocol needs to be developed for the deposition of these antibodies onto the cartridge. This is a very lengthy project that can include optimisation steps for several different aspects (i.e. coating buffers, incubation times, reagent compatibility, and compatibility with the material of the electrode). By creating a fixed array with anti-tags, these only need to be optimised once and can be reused depending on the target analyte. For example, if an array consists of 4 anti-tags, then the optimisation for the assay will only involve how much of the capture antibody is required for the assay, after it is being conjugated on the respective tag. If another panel of analytes is required, then we only need to change the antibody and re-conjugate the appropriate tag to it. This can significantly reduce assay development time and greatly facilitate the manufacturing of microfluidic - based cartridge systems.

### FIGURES

Figure 1a - Basic two site sandwich assay
Figure 1b - Basis two site sandwich assay with tag/anti-tag
Figure 2a - Competitive immunoassay
Figure 2b - Competitive immunoassay with anti-tag separation
Figure 3a - Competitive (inverted) immunoassay
Figure 3b - Competitive (inverted) immunoassay with anti-tag separation
Figure 4a - Direct immunoassay for patient antibody detection
Figure 4b - Direct immunoassay with anti-tag separation
Figure 5a - Indirect immunoassay for patient antibody detection
Figure 5b - Indirect immunoassay for patient antibody detection with anti-tag separation

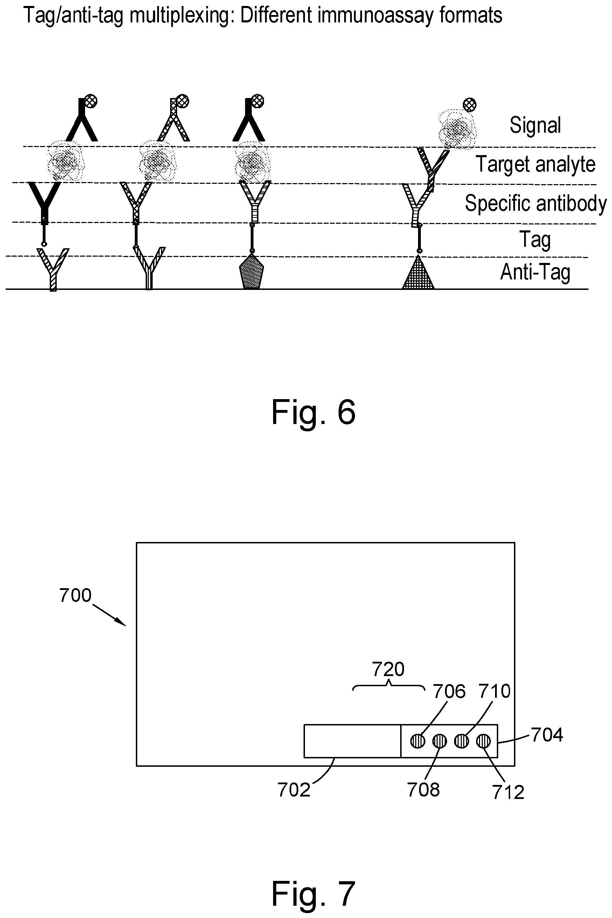

Figure 6 - Tag/anti-tag multiplexing: Different immunoassay formats This Figure provides an example of how different immunoassay formats can be combined in a single device/method using an array of different anti-tags
Figure 7 - Device comprising a multiplex sensor

Brief description: A device **700** comprising a multiplex sensor **720.** The multiplex sensor including a sensor chamber **702** for incubation of a sample with tag reagents which can then flow or be fluidically driven to a detection zone **704** comprising an array of different anti-tags **(706, 708, 710, 712).**

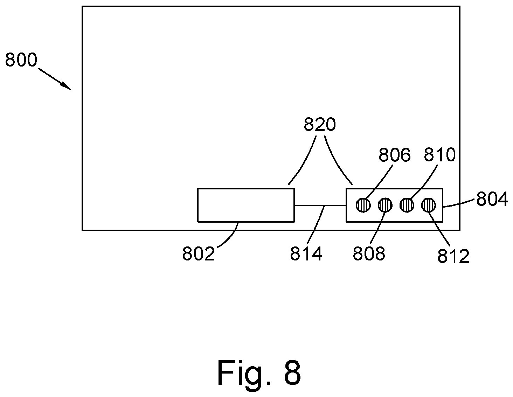

Figure 8 - Device comprising an alternative multiplex sensor.

Brief description: A device **800** comprising a multiplex sensor **820.** The multiplex sensor including a microfluidic delay channel **814** between the sensor chamber **802** and the detection zone **804.**

### DETAILED DESCRIPTION OF THE INVENTION

### Immunoassays

An immunoassay is a biochemical test that measures the presence or concentration of a macromolecule or a small molecule in a solution through the use of an antibody or an antigen. Antibodies have the potential of binding to an antigen, which itself could also be an antibody and newer developments have seen the deployment of other binding reagents such as Molecular Imprinted Polymers, Affimers, Aptamers and antibody fragments. From here on in, these are all referred to as "antibody or antibodies". The molecule detected by the immunoassay is often referred to as an analyte and is in many cases a protein, although it may be other kinds of molecules, of different size and types, as long as the proper antibodies that have the adequate properties for the assay are developed. Analytes in biological liquids such as serum or urine are frequently measured using immunoassays for medical and research purposes.

Immunoassays are often used for the detection of a specific analyte within a sample. There are a number of immunoassay formats. For example, five different common immunoassay formats are shown in Figures 1-5 (parts A) which all rely on the separation of antibody-bound analyte and free (non-bound) antibody. Variations of these five immunoassay formats with anti-tag separation of a tagged immune complex are shown in Figures 1-5 (parts B).

Basic two site sandwich assay (Figure 1A): This typical sandwich assay uses a primary immobilised capture antibody and a secondary signal antibody to bind to different sites of an analyte. These sandwich assays rely on the immobilisation of the capture antibody and removal of excess signal antibody before readings are taken. Signal generated is proportional to the amount of analyte sandwiched. No target analyte results in signal noise only. Sandwich assays are used for example in pregnancy test devices.

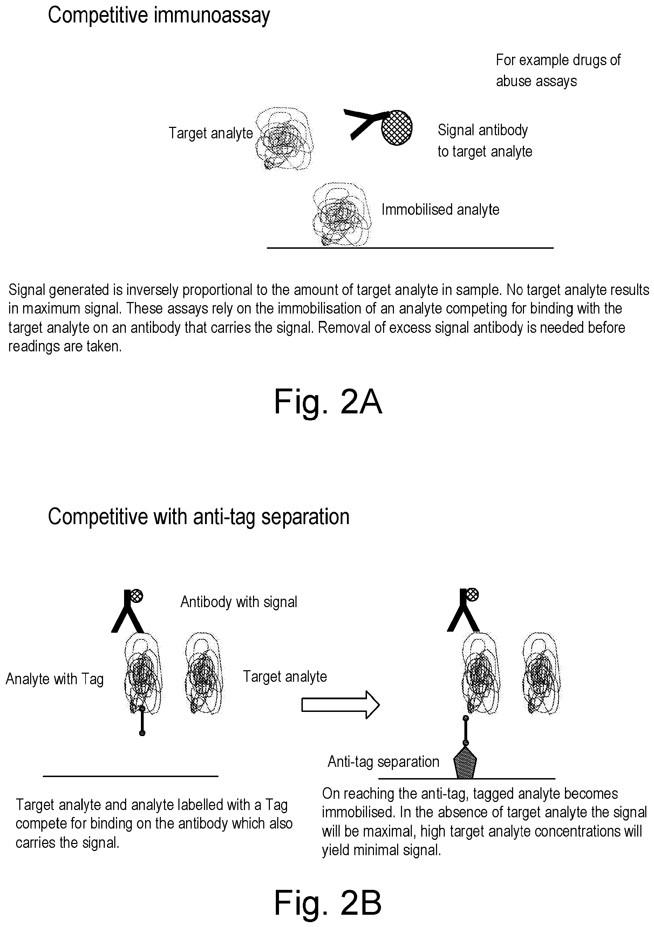

Competitive immunoassay (Figure 2A): These assays rely on the immobilisation of an analyte competing for binding with the target analyte on an antibody that carries a signal. Removal of excess signal antibody is needed before a reading is taken. Signal generated is inversely proportional to the amount of target analyte in the sample. No target analyte results in maximum signal. Competitive immunoassays are used for example in drugs of abuse assays.

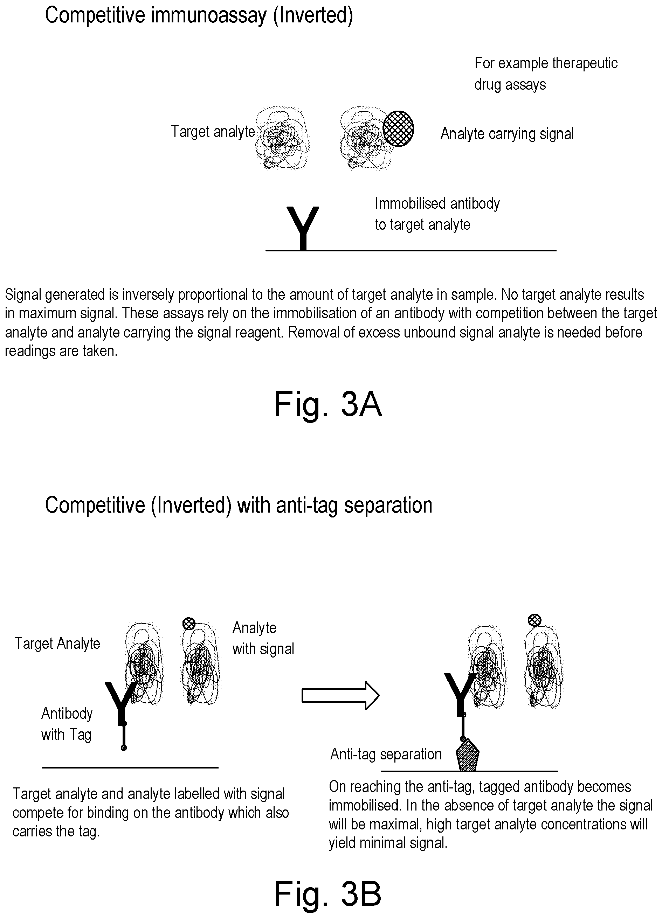

Competitive (inverted) immunoassay (Figure 3A): These assays rely on the immobilisation of an antibody with competition between the target analyte and analyte carrying the signal reagent. Removal of excess unbound signal analyte is needed before readings are taken. Signal generated is inversely proportional to the amount of target analyte in the sample. Competitive (inverted) immunoassays are used for example in therapeutic drug assays.

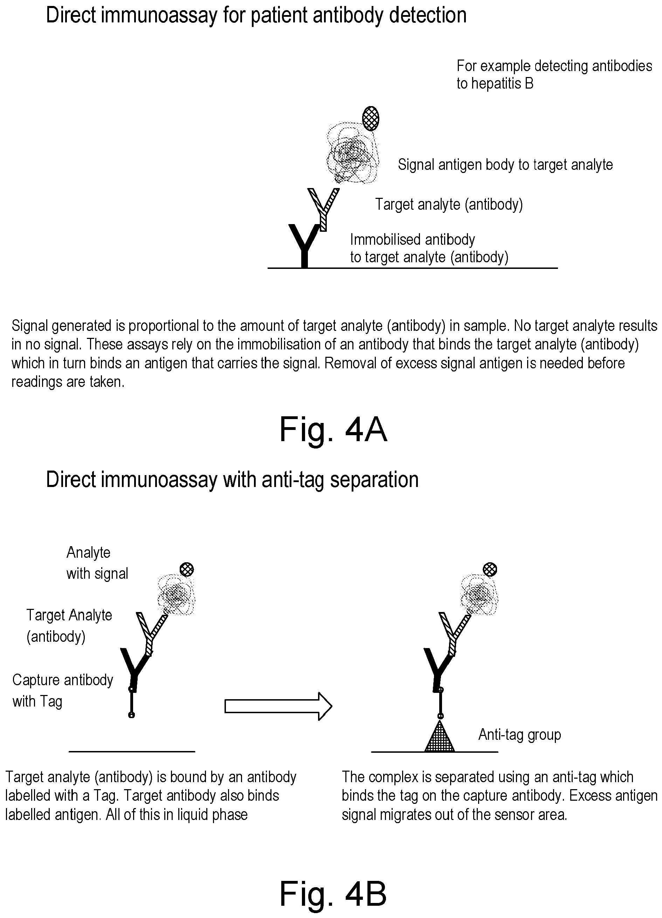

Direct immunoassay for detecting patient antibodies (Figure 4A): These assays rely on an immobilisation of an antibody that binds the target analyte (antibody) which in turn binds an antigen that carries the signal. Removal of excess signal antigen is needed before readings are taken. Signal generated is proportional to the amount of target analyte (antibody) in sample. No target analyte results in no detectable signal. Direct immunoassays for detecting patient antibodies are used for example in detecting antibodies to Hepatitis B.

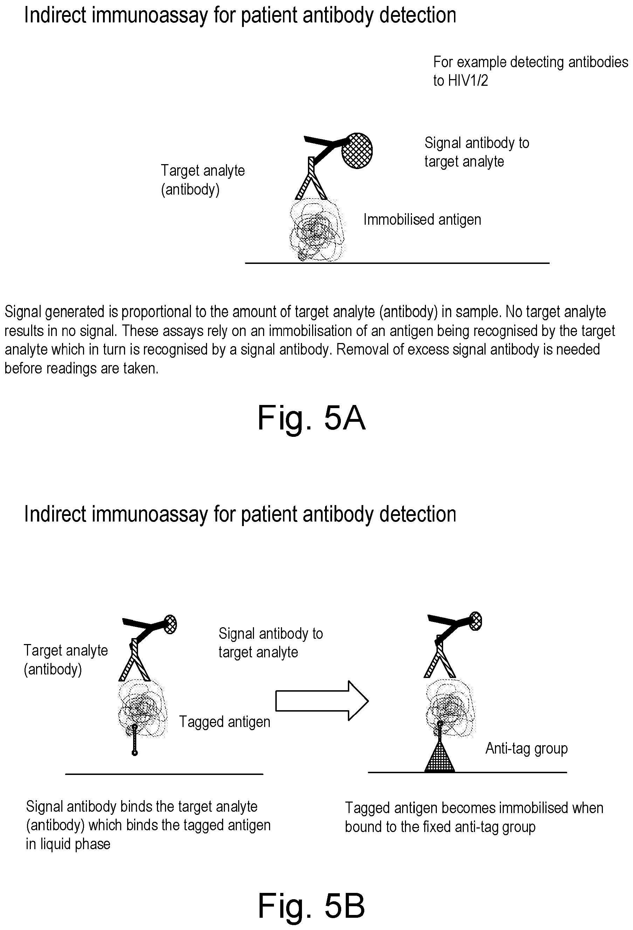

Indirect immunoassay for detecting patient antibodies (Figure 5A): These assays rely on an immobilisation of an antigen being recognised by the target analyte (antibody) which in turn is recognised by a signal antibody. Removal of excess signal antibody is needed before readings are taken. Signal generated is proportional to the amount of target analyte (antibody) in sample. Indirect immunoassays for detecting patient antibodies are used for example in detecting antibodies to HIV1/2.

These standard assays are shown in Figures 1 to 5 (parts A). Variations of each of these assay formats with anti-tag separation (comprising tag/anti-tags) are shown in Figures 1 to 5 (parts B). The present inventors have developed a tag/anti-tag system for multiplex assays involving anti-tag separation methods as illustrated in Figures 1 to 5 (part B) and described below.

Basic two site sandwich assay with tag/anti-tag (Figure 1B): Capture antibody carries a tag and is specific for the target analyte. Signal antibody is also specific for the target analyte and both antibodies bind the target as it migrates through the device. An immobilised anti-tag that binds to the tag effectively removes the tagged complex from the liquid phase as it comes into contact with it. Unreacted signal antibody will flow away from the sensor or can be washed away.

Competitive immunoassay with anti-tag separation (Figure 2B): Target analyte and analyte labelled with a Tag compete for binding on the antibody which also carries the signal. On reaching the anti-tag, tagged analyte becomes immobilised. In the absence of target analyte the signal will be maximal, whereas high target analyte concentrations will yield minimal signal.

Competitive (inverted) immunoassay with anti-tag separation (Figure 3B): Target analyte and analyte labelled with signal compete for binding on the antibody which also carries the tag. On reaching the anti-tag, tagged antibody becomes immobilised. In the absence of target analyte the signal will be maximal, whereas high target analyte concentrations will yield minimal signal.

Direct immunoassay with anti-tag separation (Figure 4B): Target analyte (antibody) is bound by an antibody labelled with a tag. Target antibody also binds labelled antigen (analyte with signal). All of this occurs in the liquid phase. The complex is separated using an anti-tag which binds the tag on the capture antibody. Excess antigen signal migrates out of the sensor area.

Indirect immunoassay for patient antibody detection with anti-tag separation (Figure 5B): Signal antibody binds the target analyte (antibody) which binds the tagged antigen in liquid phase. Tagged antigen becomes immobilised when bound to the fixed anti-tag.

Capture antibodies and signal antibodies for use in the immunoassays described herein are well-known in the art. As are the labelling techniques used in the immunoassay formats to provide signal antigen or signal analyte.

### Multiplex assay

A multiplex assay is a type of assay used to simultaneously measure multiple analytes in a single run/cycle of the assay. The conventional multiplex approach requires the deposition of individual binding reagents (antibodies or antigens) in a fixed array to make a test panel. This requires complex equipment & processes to deliver either on continuous reel systems or discreet dispensing into a device array. Increasing the number of analytes in a panel increases the risk of batch failure since only one assay has to fall out of specification for the entire panel to fail.

A multiplex device and assay methods provided herein use multiple tags/anti-tags. In particular, they use an array of different anti-tags which are immobilised to a solid support e.g. surface of a sensor. Each anti-tag can bind a complimentary tag and so is able to immobilise a complimentarily tagged antibody or complimentarily tagged antigen, thereby separating tagged immune complexes, that have formed in the liquid phase, from the liquid phase.

In some embodiments, there is a sensor chamber for incubation of a sample with tag reagents so that tagged immune complexes form in the liquid phase which can then flow or be fluidically driven to a detection zone comprising an array of different anti-tags. The sample may also interact with signal reagents (e.g. labelled antibodies or antigens) in the sensor chamber to facilitate detection.

In some embodiments, the flow of a fluid through a microfluidic device may be controlled and/or manipulated by an external means such as applying pressure to a fluid channel using an actuator such as a flow pump. The pump used to drive a fluid through the microfluidic device may be an electrically-driven pump, a thermally-driven pump or a pneumatically-driven pump. The use of a pump may be advantageous as it enable the precise control of a flow rate through the microfluidic device.

Additionally or alternatively, the flow of a fluid through a microfluidic device may be provided via capillary action. Capillary action or motion can be advantageous as it may enable a fluid to flow through the microfluidic device without the requirement of providing any external forces, such as pressure provided by a pump.

In some embodiments, a valve may be provided within the channels of the microfluidic device to control the flow rate of a fluid flow through the microfluidic device. In some embodiments, the channels of the may be provided with one or more resistors, which may be useful in controlling the flow rates of a fluid through the microfluidic device.

There may be multiple reaction zones for tagged immune complexes to form in the liquid phase, wherein reaction zone has different anti-tag.

Example 4 describes several different experimental setups for multiplexed assays in a cardiac panel which use basic two site sandwich assays e.g. with different detection methods and/or devices. The same array of anti-tags can be used to detect other target analytes (i.e. other than cardiac markers), and with other immunoassay formats. Other sets of anti-tags can be devised and tailored to the intended use. Though the invention advantageously enables the provision of an array of anti-tags that can be used for multiple tests.

In one embodiment, described in example 4E, there is a microfluidic device and method for detecting multiple target analytes in a test liquid sample using a set of complimentary tags and anti-tags and impedance spectroscopy. The detection zone of the device comprises an array of electrodes (e.g. 6), wherein there is a different anti-tag (e.g. antibodies raised against FITC, Flag, His-6, c-Myc, V5 and GST) on each electrode, and wherein each anti-tag is adsorbed or chemically linked to the electrode (e.g. wherein the electrodes are gold electrodes). The test liquid sample (e.g. serum or plasma) is added to a sensor chamber upstream of the electrodes that already contains the tag and signal reagents (e.g. tagged primary and secondary antibodies conjugated to HRP). After sufficient incubation time allowing for the formation of specific sandwich complexes with each target analyte, the mixture is fluidically driven to the anti-tag coated electrodes of the sensor and tagged immune complexes bind to their respective anti-tags. After sufficient incubation time, a wash step is introduced to remove any unbound excess material (antibody or antigens). Precipitating solution (e.g. DAB) is added to the sensor for a controlled period of time and reacts with signal reagent present in the immune complex (e.g. HRP conjugated to secondary antibody) to produce an insulating precipitate on the electrode surface. The quantity of the precipitate (which is proportional to the amount of HRP-tagged secondary and thus proportional to the amount of antigen) can be measured electrochemically using standard faradaic impedance spectroscopy using a redox probe solution. In this, the magnitude of the charge transfer resistance, Rct, at the half-wave potential of a dissolved reversible redox probe is measured by fitting an impedance spectrum to a suitable Equivalent circuit. Rct will be proportional to the amount of the insulating precipitate created on the surface. Alternatively, other parameters derived from the impedance response may be used to transduce the signal.

### Target analytes

The target analytes comprise antibodies and/or antigens.

Immunoassays rely on the ability of an antibody to recognize and bind a specific macromolecule in what might be a complex mixture of macromolecules. In immunology the particular macromolecule bound by an antibody is referred to as an antigen and the area on an antigen to which the antibody binds is called an epitope.

In some cases, an immunoassay may use an antigen to detect for the presence of antibodies, which recognize that antigen, in a solution. In other words, in some immunoassays, the analyte may be an antibody rather than an antigen.

### Sample

The sample is a liquid sample and can be any test solution. For example, blood, serum, plasma, cell and tissue lysates, or urine. The sample can be from a human, mammal or other animal, or the sample can be of a non-mammalian nature e.g. bacterial proteins. The sample is suitably chosen for human clinical testing or veterinary testing. However, the devices and methods provided herein can also be applied to other samples for other applications of immunoassay e.g. in the Agri-chemical business (e.g. testing for fungal residues and fungicides in fruit, pesticides in water or mycotoxins in grains/coffee as well as the testing of hormones and antibiotics in milk are typical examples).

### Solid surface

The solid surface on which the multiple different anti-tags are immobilised, is suitably the surface of a sensor. The sensor surface can comprise a metallic surface or metallic film, polymeric surface and in general conductive or non-conductive materials. However, the surface of the sensor comprises either i) the surface of an electrode/conductive structure itself or ii) the conductive or semiconductive coating of a non-conductive material (to function as an electrode).

The anti-tags can be adsorbed or chemically linked the surface of the sensor e.g. a metal surface or an electrode surface. In some embodiments, the sensor comprises multiple electrodes and there is a different anti-tag on each electrode. In one example, the sensor may be composed of one or more electrodes such as gold electrodes or silver/silver chloride electrodes or BST electrodes. By providing a sensor with one or more electrodes on its surface, the sensor can be suitably modified for electrochemical impedance measurements. In some embodiments, the sensor may comprise a reference electrode, a working electrode and a counter electrode, whereby a current flowing between the counter electrode and the working electrode can be continuously monitored.

The substrate (surface) of the electrode may comprise any electrically conducting material. The electrode surface may comprise a metal or carbon. The metal may be a metal in elemental form or an alloy of a metal. Optionally, the whole of the electrode surface comprises a metal or carbon. The electrode surface may comprise a transition metal. The electrode surface may comprise a transition metal selected from any of groups 9 to 11 of the Periodic Table. The surface may comprise a metal selected from, but not limited to, rhenium, iridium, palladium, platinum, copper, indium, rubidium, silver and gold. The surface may comprise a metal selected from gold, silver and platinum. The surface may comprise a carbon-containing material, which may be selected from edge plane pyrolytic graphite, basal plane pyrolytic graphite, glassy carbon, boron doped diamond, highly ordered pyrolytic graphite, carbon powder and carbon nanotubes.

Alternatively, the anti-tags can be adsorbed or chemically linked to the surface of the sensor comprising a non-conductive plastic surface, if this surface is coated with a conductive or semiconductive polymeric material. In some embodiments the sensor surface comprises a conductive polymer, e.g. PEDOT - polystyrene sulfonate or poly(3,4-ethylenedioxythiophene), PANI (polyanilines), and/or PPY (poly(pyrrole)s). It is known in the art to coat plastic surfaces with conductive polymers such as PEDOT to function as electrodes.

### Anti-tags/Tags

Provided herein is an array of different anti-tags immobilised to a solid surface. Each anti-tag is able to bind a complimentary tag. The anti-tag may be a group (e.g. antibody fragments) or species.

Epitope tags are used extensively in proteomics & protein chemistry and can be an amino acid sequence or molecule attached to or assembled into a target analyte (protein or nucleic acid). The matching binding reagent - the anti-tag is typically an antibody but could also be an aptamer, affimer, molecularly imprinted polymer (MIP) or other binding reagent, or nucleic acid(s) or antibody fragments. Other suitable binding reagents include: Nucleic acids (oligonucleotides), peptides (e.g.flag tags), enzymes and chemicals (e.g. FITC). The most common tag/anti-tag system is biotin-avidin but there are many options available (e.g. see Table 1). Typically, anti-tags are antibodies with good affinity.

**Table 1: Example tag/anti-tag pairs**

| **Tag** | **Anti-Tag antibody** |
|---|---|
| Primary ab e.g. mouse | Sheep anti-mouse ab |
| Biotin | Anti-biotin (or avidin) |
| FITC | Anti-FITC |
| HRP | Anti-HRP |
| R Maltose Binding Protein | Anti-MPB |
| Streptavidin | Anti-Streptavidin (or biotin) |
| Flag | Anti-Flag |
| His-6 | Anti-His-6 |
| c-Myc | Anti-c-Myc |
| biotin acceptor peptide | Anti-biotin acceptor peptide |
| V5 | Anti-V5 |
| glutathione S-transferase (GST) | Anti- glutathione S-transferase (GST) |

In a device or method provided herein, any of the anti-tags shown in Table 1, or other tag/anti-tag pairs, may be combined to form a panel/array of different anti-tags. The set of tags/anti-tags are tailored to the signal measurement method used e.g. anti-FITC/FITC would not be selected as an anti-tag/tag pair for use with fluorometric detection, and anti-HRP/HRP would not be selected as an anti-tag/tag pair for use with colorimetric detection.

In some embodiments the multiplex assay comprises 3-10 different tag/anti-tag pairs. In some embodiments, the panel of anti-tags comprises 6 different anti-tags, e.g. antibodies against FITC, Flag, His-6, c-Myc, V5 and GST; or e.g. antibodies against biotin, Flag, His-6, c-Myc, V5 and GST.

The immunoassay formats with tag/anti-tag separation as shown in Figures (1-5 part B) provided herein, use tagged antibody or tagged competing analyte and immobilised anti-tag.

It would be understood that each tag/anti-tag binding pair could be used in any suitable configuration. For example, the tag could be immobilised and the anti-tag chemically attached to the antibody or antigen as well as the configuration of immobilised anti-tag that is described herein. Any combination of the anti-tag/tag binding pairs can be deployed to create a suitable configuration for multiplexed assays.

### Signal / Signal measurement technique

In addition to the binding of an antibody to its antigen, the other key feature of all immunoassays is a means to produce a measurable signal in response to the binding. Most, though not all, immunoassays involve chemically linking antibodies or antigens with some kind of detectable label. A large number of labels exist in modern immunoassays, and they allow for detection through different means. Many labels are detectable because they either emit radiation, produce a color change in a solution, fluoresce under light, or can be induced to emit light.

In some embodiments, the multiplex assay comprises a signal antibody or signal antigen/analyte. For examples, Figures 1, 2 and 5 show use of a signal antibody (antibody carrying a signal/label) and Figures 3 and 4 show use of a signal antigen (antigen carrying a signal/label). Some embodiments utilise colorimetric detection or fluorometric detection, e.g. see examples 4A and 4B respectively. Some embodiments utilise FRET (Fluorescence resonance energy transfer), e.g. see example 4G.

Some embodiments utilise impedance measurement or redox active precipitate for detection. See examples 4C and 4D. In these examples, secondary antibodies (that can bind the target analytes) are conjugated with HRP and a precipitating solution (e.g. DAB, TMB, AEC) which reacts with HRP is added to the sensor to produce a precipitate.

In some embodiments, the assay methods provided herein utilise electrical impedance spectroscopy methods (e.g. faradaic or non-faradaic impedance spectroscopy) to determine the presence and/or amount of analytes of interest. In this case, the anti-tags are immobilised at fixed points on the sensor surface suitable for impedance measurement (e.g. electrode surface). In these embodiments, a precipitating solution is added to produce an insulting precipitate on an electrode surface. The quantity of the precipitate (which is proportional to the amount of target analyte) can be measured electrochemically using standard faradaic impedance spectroscopy. In this, the magnitude of the charge transfer resistance, Rct, at the half-wave potential of a dissolved reversible redox probe is measured by fitting an impedance spectrum to a suitable Equivalent circuit. Rct will be proportional to the amount of the insulating precipitate created on the surface. Alternatively, other parameters derived from the impedance response may be used to transduce the signal. See Example 4C.

In some embodiments, the quantity of the precipitate (which may be proportional to the amount of HRP-tagged secondary and thus proportional to the amount of antigen) can be measured electrochemically using any range of electrochemical techniques which include chronoamperometry, cyclic voltammetry, pulsed voltammetric methods such as Different Pulsed Voltammetry (DPV) and Square Wave Voltammetry (SWV), and potentiometric methods such as Open Circuit Potential (OCP) or chronopotentiometry. For example, the amount of the precipitate can be measured using DPV by scanning from low to high potential in an inert electrolyte and measuring the peak oxidative current generated as the precipitate (initially in reduced form at low potentials) is re-oxidised.

Precipitating substrates that may be used in the assay methods described herein are set out in table 2 below. Precipitating substrates are typically used for western blot and immunohistochemistry detection and are, therefore, well known in the literature and available for use in the present assay methods.

**Table 2: Precipitating chromogenic substrates for HRP and AP.**

| Substrate | Format | Enzyme | Features | Relative sensitivity | Signal colour |
|---|---|---|---|---|---|
| DAB | Dry powder | HRP | Can be formulated | Medium | Brown |
| Metalenhanced DAB | 2-component reagent kit | HRP | 50X more sensitive than DAB alone | Highest | Brown to black |
| BCIP | Dry powder | AP | Can be formulated | Medium | Blue to purple |
| NBT | Dry powder | AP and glucose oxidase | Non-carcinogenic | Medium | Blue to purple |
| 1-step NBT/BCIP | Single step, ready-to-use | AP | Low background for high sensitivity | High | Black to purple |
| 1-step NBT/BCIP + suppressor | Single step, ready-to-use | AP | May or may not contain levamisole for endogenous alkaline phosphatase inhibition | High | Black to purple |
| TMB | Single step, ready-to-use | HRP | Very high sensitivity and stability | high | Blue |
| AEC | Single step, ready-to-use | HRP | Ready to use moderate stability | low | Orange-brown |
| Vector^{®} Black | 3 component reagent | AP | Very high sensitivity | high | Black |
| Vector^{®} NovaRED | 4 component reagent | HRP | Very high sensitivity | high | red |

### Examples

### Example 1 - Universal platform

Consider a multiplex panel for infectious disease testing looking to detect human IgG antibodies to toxoplasma, CMV, HSV1, HSV1, measles, mumps & rubella. This could use a direct immunoassay format and each chemistry would require the immobilisation of its respective antibody onto a surface in a fixed array suitable for reading the signal. This process is typically done as a large batch on a dedicated pipetting/dispensing system.

Large scale manufacturing a diverse range of multiplex tests by depositing specific antigens or antibodies that could also include allergy testing, therapeutic drugs, cardiac markers, blood borne viruses and hormones would create either a bottleneck in production or the need for a many dispensing systems.

By using multiple tag/anti-tags, a single universal platform could be utilised for all the different multi-panel tests. This would require just one sensor platform with 6 different anti-tags attached in an array. Selectively adding different tags to the selected antibody or antigen for a given panel and adding these as a separate downstream reagent would complete the device.

As an example, Table 3 below shows the same universal platform with anti-tags (1-6) fixed above the sensor area. For Rubella within the infectious disease panel using an indirect format, each rubella antigen would be labelled with tag 1 and a common signal antibody of say anti-human IgG HRP conjugate added. Human IgG to rubella would bind to the antigen and also be bound by the signal antibody. The immobilised anti-tag 1 would extract the complex onto the sensor surface. This would be repeated using different tag/anti-tags (2-6) for the measles, mumps etc

Using the same universal platform with the same array of anti-tags, other test panels can be created by substituting in the relevant chemistries.

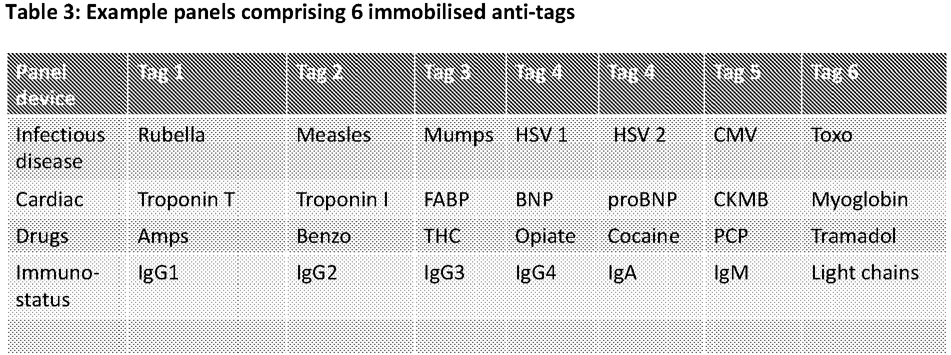

This is analogous to the manufacturing processes used in the car industry: The Volkswagen Group use a common platform (known as MQB) that covers over a dozen different cars (Audi TT, Seat Ateca, Skoda Octavia and VW Tiguan to name a few).

Within many areas of testing there could easily be anywhere from 10 to 100 different target analytes of interest depending on the market and this often requires subtle changes to the panels offered. For example there are at least 14 different drugs of abuse that could be tested for but typically this is restricted to 2 or 3 variations of 6-panel tests: they tend to be created if substantial market demand and volume dictates. The number of 6-panel permutations from 14 drugs could yield over 3000 different 6-panel tests which would not be economically viable. Smaller volume requests for different 6-panel tests could be achieved using the greater flexibility, choice and speed to market of the universal platform.

Allergy testing is another example where specific combinations of tests could be required from the many allergens within a class.

### Example 2 - Faster kinetics

Antibody and antigens bind quickly in liquid phase, the strength of the binding is called the affinity. Affinities can be described as weak, medium or strong. Immobilising one or other of these on a solid surface reduces the opportunity of antibody-antigen interaction and as such reduces the binding kinetics. To compensate for this incubation times are increased when one moiety is immobilised on a solid phase.

Using the tag/anti-tag format, the primary incubation between target specific antibodies and antigens happens in liquid phase. This benefits weak to medium affinity reactions as well as detecting low concentrations (free movement of molecules allows greater opportunity of meeting up). Detecting low concentration analytes can be improved by lengthening the liquid phase incubation, this can be achieved in the sensor by moving these key reagents further away from the sensor. For example, including a microfluidic delay channel into the device can increase incubation time.

Separation of the liquid phase immune complex using tag/anti-tag also relies on the binding affinity of the tag/anti-tag. These can be used in high concentration and be selected to have medium to high binding affinity. This allows for relatively short incubation times.

Primary incubation in the liquid phase allows for incubation time to be varied, and allows for a more automated approach to multiplexed assays.

### Example 3 - Mixed mode immunoassay

One of the reasons different immunoassay formats have evolved has been driven by the need to measure at greater analytical sensitivity. As a general rule sandwich assays tend to have greater sensitivity than competitive assays. Sandwich assays are not typically used to detect small molecules such as drugs and steroid hormones (though a sandwich format can be potentially be used by either using other species (aptamers) or indirectly by targeting metabolic products of drugs/hormones).

In some circumstances a multiplex panel needs to include analytes that span a wide range in analytical concentration and include small and large molecules. For example sports medicine testing could involve simultaneous monitoring of low levels of cortisol (0.00003 to 0.0003g/L) whilst also quantitating higher levels of Immunoglobulin G (8 to 16 g/L) and Immunoglobulin A (1 to 4 g/L). Testosterone and Immunoglobulin M might also be included in such a panel. The ability to simultaneously run different formats on the same panel, without significantly altering the dilution of the sample, is a good benefit.

Other scenarios might involve the detection of an anti-cancer drug whilst measuring various tumour markers.

### Example 4 - Experimental set-ups

### A) Experimental setup using the Tag/anti-tag system for multiplexed assays in a cardiac panel using colorimetric detection

In this setup, a panel of 6 antibodies raised against FITC, Flag, His-6, c-Myc, V5 and GST tags are adsorbed or chemically linked to a plastic or metal surface. These are part of an array (sensor) that is composed of 6 electrodes. Capture (primary) antibodies specific to several different markers e.g. CRP, Tnl, TnT, D-Dimer, BNP and proBNP, each are conjugated with one of the above tags using available coupling chemistry conjugation kits (EDC/NHS). A set of secondary antibodies are selected against the targets mentioned above and are conjugated to HRP. Sample, either serum or plasma is being added to a sensor chamber that is before the electrodes and already contains the tagged primary and secondary antibodies. Sufficient incubation time allows for the formation of specific sandwich complexes with each target analyte. Afterwards the mixture is being fluidically driven to the anti-tag coated electrodes of the sensor. Another binding event takes place, with each of the capture antibody, containing a specific tag e.g. His-6, binding to the respective anti-his-6 antibody on the surface of the electrode. After sufficient incubation time, a wash step in introduced to remove any unbound excess material (antibody or antigens). A chromogenic solution, (e.g. TMB) is added to the sensor and reacts with the HRP to produce a characteristic blue colour. The intensity of the colour can be measured colorimetrically at 605nm after a specific reaction time. The signal intensity is proportional the target analyte.

### B) Experimental setup using the Tag/anti-tag system for multiplexed assays in a cardiac panel using fluorometric detection

In this setup, a panel of 6 antibodies raised against biotin, Flag, His-6, c-Myc, V5 and GST tags are adsorbed or chemically linked to a plastic or metal surface. These are part of an array (sensor) that is composed of 6 detection zones. Capture (primary) antibodies specific to several different markers e.g. CRP, Tnl, TnT, D-Dimer, BNP and proBNP, each are conjugated with one of the above tags using available coupling chemistry conjugation kits (EDC/NHS). A set of secondary antibodies are selected against the targets mentioned above and are conjugated with different fluorogenic molecules. The secondary anti-CRP and anti-Tnl antibodies can be conjugated with FITC, the secondary anti-TnT and anti D-Dimer with Alexa Fluor 750 and the secondary anti-BNP and anti-proBNP antibodies with Cy7.5. The selection of these dyes also offers greater flexibility as they have distinct excitation/emission wavelengths. Sample, either serum or plasma is being added to a sensor chamber that is before the detection zones and already contains the tagged primary and secondary antibodies. Sufficient incubation time allows for the formation of specific sandwich complexes with each target analyte. Afterwards the mixture is being fluidically driven to the anti-tag coated electrodes of the sensor. Another binding event takes place, with each of the capture antibody, containing a specific tag e.g. His-6, binding to the respective anti-his-6 antibody on the surface of the electrode. After sufficient incubation time, a wash step in introduced to remove any unbound excess material (antibody or antigens). Detection is performed by use of a fluorometer using the corresponding excitation/emission wavelengths. Signal intensity is proportional to the bound analyte.

### C) Experimental setup using the Tag/anti-tag system for multiplexed assays in a cardiac panel using faradaic or non-faradaic impedance spectroscopy

In this setup, a panel of 6 antibodies raised against FITC, Flag, His-6, c-Myc, V5 and GST tags are adsorbed or chemically linked to a gold electrode. These are part of an array (sensor) that is composed of 6 electrodes. Capture (primary) antibodies specific to several different markers e.g. CRP, Tnl, TnT, D-Dimer, BNP and proBNP, each are conjugated with one of the above tags using available coupling chemistry conjugation kits (EDC/NHS). A set of secondary antibodies are selected against the targets mentioned above and are conjugated with HRP. Sample, either serum or plasma is being added to a sensor chamber that is before the electrodes and already contains the tagged primary and secondary antibodies. Sufficient incubation time allows for the formation of specific sandwich complexes with each target analyte. Afterwards the mixture is being fluidically driven to the anti-tag coated electrodes of the sensor. Another binding event takes place, with each of the capture antibody, containing a specific tag e.g. His-6, binding to the respective anti-his-6 antibody on the surface of the electrode. After sufficient incubation time, a wash step in introduced to remove any unbound excess material (antibody or antigens). Precipitating solution (e.g. DAB) is added to the sensor for a controlled period of time and reacts with the HRP to produce an insulating precipitate on the electrode surface. The quantity of the precipitate (which is proportional to the amount of HRP-tagged secondary and thus proportional to the amount of antigen) can be measured electrochemically using standard faradaic impedance spectroscopy. In this, the magnitude of the charge transfer resistance, Rct, at the half-wave potential of a dissolved reversible redox probe is measured by fitting an impedance spectrum to a suitable Equivalent circuit. Rct will be proportional to the amount of the insulating precipitate created on the surface. Alternatively, other parameters derived from the impedance response may be used to transduce the signal.

### D) Experimental setup using the Tag/anti-tag system for multiplexed assays in a cardiac panel using redox active precipitate

In this setup, a panel of 6 antibodies raised against FITC, Flag, His-6, c-Myc, V5 and GST tags are adsorbed or chemically linked to a gold electrode. These are part of an array (sensor) that is composed of 6 electrodes. Capture (primary) antibodies specific to several different markers e.g. CRP, Tnl, TnT, D-Dimer, BNP and proBNP, each are conjugated with one of the above tags using available coupling chemistry conjugation kits (EDC/NHS). A set of secondary antibodies are selected against the targets mentioned above and are conjugated with HRP. Sample, either serum or plasma is being added to a sensor chamber that is before the electrodes and already contains the tagged primary and secondary antibodies. Sufficient incubation time allows for the formation of specific sandwich complexes with each target analyte. Afterwards the mixture is being fluidically driven to the anti-tag coated electrodes of the sensor. Another binding event takes place, with each of the capture antibody, containing a specific tag e.g. His-6, binding to the respective anti-his-6 antibody on the surface of the electrode. After sufficient incubation time, a wash step in introduced to remove any unbound excess material (antibody or antigens). A solution of precipitating solution capable of producing a redox active precipitate (e.g TMB, DAB, AEC) is added to the sensor and reacts with the HRP to produce the redox active precipitate on the electrode surface. The quantity of the precipitate (which is proportional to the amount of HRP-tagged secondary and thus proportional to the amount of antigen) can be measured electrochemically by several conventional techniques, including amperometry (measuring the charge transferred to the precipitate), voltammetry or impedance (by measuring the ability of the precipitate to capacitate charge at its half-wave potential).

### E) Experimental setup using the Tag/anti-tag system for multiplexed assays for a cardiac panel in a microfluidic device.

In this setup, a panel of 6 antibodies raised against FITC, Flag, His-6, c-Myc, V5 and GST tags are adsorbed or chemically linked to a gold electrode for 1 hour in a concentration of 1ug/ul. These are part of an array (sensor) that is composed of 6 electrodes. Capture (primary) antibodies specific to several different markers e.g. CRP, Tnl, TnT, D-Dimer, BNP and proBNP, each are conjugated with one of the above tags using available coupling chemistry conjugation kits (EDC/NHS). A set of secondary antibodies are selected against the targets mentioned above and are conjugated with HRP. 100 ul of whole blood are added in the slot of a microfluidic cartridge and around 50 ul of plasma are separated using a plasma separation membrane. Then the plasma is diluted to an appropriate concentration using a sample diluent. The diluted samples is then microfluidically driven into a chamber containing the capture and detection antibody either in a dried format or in liquid form in concentrations of 0.5 ng/ml. After 5 min of incubation the mixture containing the sandwich complexes of the antibodies with the antigens is further incubated for 4 min on the 6 electrodes of the sensor. Each electrode depending on the tag it contains will bind to the respective sandwich complex from the previous incubation. After the incubation is completed, the sensor chamber is flushed with 200ul wash buffer and incubated for further 2 min. Finally, 100 ul of precipitating chromogenic solution (DAB, TMB) are added into the sensor chamber and incubated for 3 min. After the incubation, the reaction is terminated by flushing the chamber with 300 ul of wash buffer. The quantity of the precipitate (which is proportional to the amount of HRP-tagged secondary and thus proportional to the amount of antigen) can be measured electrochemically using standard faradaic impedance spectroscopy using 100ul of redox probe solution. In this, the magnitude of the charge transfer resistance, Rct, at the half-wave potential of a dissolved reversible redox probe is measured by fitting an impedance spectrum to a suitable Equivalent circuit. Rct will be proportional to the amount of the insulating precipitate created on the surface. Alternatively, other parameters derived from the impedance response may be used to transduce the signal.

### F) Experimental setup using the Tag/anti-tag system for multiplexed assays for a cardiac panel in a lab setup

In a high binding 96 well ELISA plate 100 ul of each of 6 antibodies raised against FITC, Flag, His-6, c-Myc, V5 and GST tags are adsorbed or chemically linked to a gold electrode for 1 hour in a concentration of 1ug/ul. After incubation, the wells are washed 3 times with PBS-T and then blocked for 30 min using a 5% BSA solution in PBS-T. In separate vials 100 ul of plasma diluted 1/1000 is being added in a vial that contains capture and HRP-labelled detection antibodies in a concentration of 0.5 ng/ml. Samples are incubated for 5 min in RT and afterwards 100 ul of the mixture is added into each of the 96 well plates. Then the samples are incubated for a further 3 min in RT. After the incubation, the wells are emptied and washed 3 times using PBS-Tween. After the wash steps, 100 ul of TMB solution is added into each well and the reaction is further incubated for 3 min in RT. The reaction is being terminated by addition of 100 ul of 2M sulfuric acid and the absorbance of each well is read at 450 nm, also using a reference wavelength at 650 nm. The change in absorbance is correlated with the concentration of each different analyte in each of the wells.

### G) Experimental setup using the Tag/anti-tag system for multiplexed assays in a cardiac panel using FRET

In this setup, a panel of 6 antibodies raised against biotin, Flag, His-6, c-Myc, V5 and GST tags are adsorbed or chemically linked to a plastic or metal surface. These are part of an array (sensor) that is composed of 6 detection zones. Capture (primary) antibodies specific to several different markers e.g. CRP, Tnl, TnT, D-Dimer, BNP and proBNP, each are conjugated with one of the above tags using available coupling chemistry conjugation kits (EDC/NHS). A set of secondary antibodies are selected against the targets mentioned above and are conjugated with different fluorogenic molecules. The secondary anti-CRP can be conjugated to RPE-APC, the anti-Tnl antibodies can be conjugated with RPE-Cy5, the secondary anti-TnT can be conjugated with RPE-Cy7, the anti D-Dimer with RPE - DyLight^{®}650, the anti-BNP with APC - DyLight755 and the anti-proBNP antibodies with Fluorescein - RPE the selection of these dyes also offers greater flexibility as they have distinct excitation/emission wavelengths and can yield distinct FRET signals. Sample, either serum or plasma is being added to a sensor chamber that is before the detection zones and already contains the tagged primary and secondary antibodies. Sufficient incubation time allows for the formation of specific sandwich complexes with each target analyte. Afterwards the mixture is being fluidically driven to the anti-tag coated electrodes of the sensor. Another binding event takes place, with each of the capture antibody, containing a specific tag e.g. His-6, binding to the respective anti-his-6 antibody on the surface of the electrode. After sufficient incubation time, a wash step in introduced to remove any unbound excess material (antibody or antigens). Detection is performed by use of a fluorometer using excitation wavelengths of the donor molecule and emission wavelengths of the donor molecule. Signal intensity is proportional to the bound analyte.

Various further aspects and embodiments of the present invention will be apparent to those skilled in the art in view of the present disclosure.

## Claims

1. A microfluidic device for conducting multiplex immunoassays to detect multiple target analytes in a liquid sample using a set of tags and anti-tags, wherein the device comprises an array of different anti-tags immobilised on a surface of a sensor for measuring a signal, wherein each anti-tag is arranged to immobilise a tagged antibody or tagged antigen thereby in use separating tagged immune complexes from the liquid phase affecting the signal measured;
wherein the device comprises a sensor chamber for tagged immune complexes to form in the liquid phase, and the device comprises one or more detection zones comprising the array of different anti-tags for immobilising tagged immune complexes;
wherein the device comprises a microfluidic delay channel between the sensor chamber and the detection zone(s) to increase the liquid phase incubation time of reagents that react to form tagged immune complexes before reaching the anti-tags; and wherein in use liquid comprising the tagged immune complexes flows or is fluidically driven from the sensor chamber to the detection zone(s).

2. The device according to claim 1, wherein the detection/signal measurement comprises colorimetric detection, fluorometric detection, faradaic or non-faradaic impedance spectroscopy, or redox active precipitate detection.

3. The device according to claim 1 or claim 2, wherein either i) a portion of the surface of the sensor comprises a conductive or semi conductive polymer or ii) the surface of the sensor comprises the surface of one or more electrodes.

4. The device according to any preceding claim, wherein the device comprises an array of electrodes with a different anti-tag on each electrode, optionally wherein each anti-tag is adsorbed or chemically linked to the electrode, and optionally wherein the electrode is a gold electrode.

5. The device according to any preceding claim, wherein the target analytes comprise antibodies and/or antigens.

6. The device according to any preceding claim, wherein each anti-tag is an antibody, aptamer, affimer or molecularly imprinted polymer (MIP).

7. The device according to claim 6, wherein when an anti-tag is an antibody, the anti-tag antibody is avidin/anti-biotin and the corresponding tag is biotin or anti-flag^{®} tag antibody against flag^{®} tag, or anti-His-6 antibody against His-6, or anti-c-myc antibody against c-myc, or anti- biotin acceptor peptide antibody against biotin acceptor peptide, or anti- V5 antibody against V5 tag or anti- glutathione S-transferase (GST) antibody against glutathione S-transferase tag, or the anti-tag antibody is sheep anti-mouse ab and the corresponding tag is primary ab e.g. mouse, or anti-FITC against FITC, or anti-HRP against HRP, or anti-MPB against R Maltose binding protein; or anti-streptavidin/biotin against streptavidin.

8. The device according to any preceding claim, wherein the device comprises an array of at least: 3 or 4 or 5 or 6 or 7 or 8 or 9 or 10 different anti-tags.

9. The device according to any preceding claim, wherein the device comprises an array of 6 different anti-tags, optionally the 6 different anti-tags are antibodies against: FITC or biotin, Flag, His-6, c-Myc, V5 and GST.

10. The device according to any preceding claim, for infectious disease testing or cardiac testing or drug testing or immunostatus/allergy testing.

11. The device according to any preceding claim, wherein the multiplex assay comprises one or more of the following immunoassay formats:
a. Two site immunometric or sandwich assay with anti-tag separation
b. Competitive immunoassay with anti-tag separation
c. Competitive immunoassay (inverted) with anti-tag separation
d. Direct immunoassay for detecting antibodies with anti-tag separation
e. Indirect immunoassay for detecting antibodies with anti-tag separation.

12. The device according to any preceding claim, wherein the array of different anti-tags is arranged to conduct immunoassays of at least two different formats on a single device.

13. The device according to claim 12, wherein the array of anti-tags is arranged to detect low levels of cortisol whilst also quantitating higher levels of immunoglobulin G and immunoglobulin A, and optionally also detecting testosterone and/or immunoglobulin M; wherein the device comprises a competitive immunoassay format for detecting cortisol and optionally testosterone, and a sandwich assay format for detecting immunoglobulins G and A and optionally immunoglobulin M.

14. The device according to claim 12, wherein the device comprises an anti-tag panel for the detection of at least one anti-cancer drug as well as various tumour markers.

## Patentansprüche

1. Mikrofluidische Vorrichtung zur Durchführung von Multiplex-Immunassays zur Detektion mehrerer Zielanalyten in einer flüssigen Probe unter Verwendung eines Satzes von Markierungen und Anti-Markierungen, wobei die Vorrichtung eine Anordnung verschiedener Anti-Markierungen umfasst, die auf einer Oberfläche eines Sensors zur Messung eines Signals immobilisiert sind, wobei jede Anti-Markierung zur Immobilisierung eines markierten Antikörpers oder markierten Antigens angeordnet ist, wodurch bei Gebrauch markierte Immunkomplexe aus der flüssigen Phase abgetrennt werden, die das gemessene Signal beeinflussen;
wobei die Vorrichtung eine Sensorkammer für markierte, in der flüssigen Phase zu bildende Immunkomplexe umfasst, und die Vorrichtung eine oder mehrere Detektionzonen umfasst, die die Anordnung verschiedener Anti-Markierungen zur Immobilisierung markierter Immunkomplexe umfassen;
wobei die Vorrichtung einen mikrofluidischen Verzögerungskanal zwischen der Sensorkammer und der/den Detektionzone(n) umfasst, um die Flüssigphasen-Inkubationszeit von Reagenzien zu verlängern, die unter Bildung von markierten Immunkomplexen reagieren, bevor sie die Anti-Markierungen erreichen; und wobei im Gebrauch Flüssigkeit, die die markierten Immunkomplexe umfasst, von der Sensorkammer zu der/den Detektionzone(n) fließt oder fluidisch getrieben wird.

2. Vorrichtung nach Anspruch 1, wobei die Detektion/Signalmessung eine kolorimetrische Detektion, eine fluorometrische Detektion, eine faradaische oder nicht-faradaische Impedanzspektroskopie oder eine redoxaktive Präzipitat-Detektion umfasst.

3. Vorrichtung nach Anspruch 1 oder Anspruch 2, wobei entweder i) ein Teil der Oberfläche des Sensors ein leitendes oder halbleitendes Polymer umfasst oder ii) die Oberfläche des Sensors die Oberfläche einer oder mehrerer Elektroden umfasst.

4. Vorrichtung nach einem der vorangehenden Ansprüche, wobei die Vorrichtung eine Anordnung von Elektroden mit einer unterschiedlichen Anti-Markierung auf jeder Elektrode umfasst, wobei gegebenenfalls jede Anti-Markierung adsorbiert oder chemisch mit der Elektrode verknüpft ist, und wobei gegebenenfalls die Elektrode eine Goldelektrode ist.

5. Vorrichtung nach einem der vorangehenden Ansprüche, wobei die Zielanalyten Antikörper und/oder Antigene umfassen.

6. Vorrichtung nach einem der vorangehenden Ansprüche, wobei jede Anti-Markierung ein Antikörper, ein Aptamer, ein Affimer oder ein molekular geprägtes Polymer (MIP) ist.

7. Vorrichtung nach Anspruch 6, wobei, wenn eine Anti-Markierung ein Antikörper ist, der Anti-Markierungs-Antikörper Avidin/Anti-Biotin ist und die entsprechende Markierung Biotin oder Anti-Flag^{®}-Tag-Antikörper gegen Flag^{®}-Tag oder Anti-His-6-Antikörper gegen His-6 oder Anti-c-myc-Antikörper gegen c-myc ist, oder Anti-Biotin-Akzeptorpeptid-Antikörper gegen Biotin-Akzeptorpeptid, oder Anti-V5-Antikörper gegen V5-Tag oder Anti-Glutathion-S-Transferase (GST)-Antikörper gegen Glutathion-S-Transferase-Tag, oder der Anti-Markierungs-Antikörper ist Schaf-Anti-Maus-ab und die entsprechende Markierung ist primäres ab e. z. B. Maus, oder Anti-FITC gegen FITC, oder Anti-HRP gegen HRP, oder Anti-MPB gegen R-Maltose-Bindungsprotein; oder Anti-Streptavidin/Biotin gegen Streptavidin.

8. Vorrichtung nach einem der vorangehenden Ansprüche, wobei die Vorrichtung eine Anordnung umfasst von wenigstens: 3 oder 4 oder 5 oder 6 oder 7 oder 8 oder 9 oder 10 verschiedene Anti-Markierungen.

9. Vorrichtung nach einem der vorangehenden Ansprüche, wobei die Vorrichtung eine Anordnung von 6 verschiedenen Anti-Markierungen umfasst, wobei es sich bei den 6 verschiedenen Anti-Markierungen gegebenenfalls um Antikörper gegen Folgendes handelt: FITC oder Biotin, Flag, His-6, c-Myc, V5 und GST.

10. Vorrichtung nach einem der vorangehenden Ansprüche zur Infektionskrankheitstestung oder Herztestung oder Arzneistofftestung oder Immunstatus/Allergietestung.

11. Die Vorrichtung nach einem der vorangehenden Ansprüche, wobei der Multiplex-Assay eines oder mehrere der folgenden Immunassay-Formate umfasst:
a. Immunometrischer Zwei-Stellen- oder Sandwich-Assay mit Anti-Markierungstrennung
b. Kompetitiver Immunassay mit Anti-Markierungstrennung
c. Kompetitiver Immunassay (invertiert) mit Anti-Markierungstrennung
d. Direkter Immunassay zur Detektion von Antikörpern mit Anti-Markierungstrennung
e. Indirekter Immunassay zur Detektion von Antikörpern mit Anti-Markierungstrennung.

12. Vorrichtung nach einem der vorangehenden Ansprüche, wobei die Anordnung verschiedener Anti-Markierungen zur Durchführung von Immunassays von wenigstens zwei verschiedenen Formaten auf einer einzigen Vorrichtung angeordnet ist.

13. Vorrichtung nach Anspruch 12, wobei die Anordnung von Anti-Markierungen zur Detektion niedriger Spiegel von Cortisol angeordnet ist, während sie auch höhere Spiegel von Immunglobulin G und Immunglobulin A quantifiziert und gegebenenfalls auch Testosteron und/oder Immunglobulin M quantifiziert; wobei die Vorrichtung ein kompetitives Immunassay-Format zur Detektion von Cortisol und gegebenenfalls Testosteron und ein Sandwich-Assay-Format zur Detektion von Immunglobulinen G und A und gegebenenfalls Immunglobulin M umfasst.

14. Vorrichtung nach Anspruch 12, wobei die Vorrichtung ein Anti-Markierungs-Panel zur Detektion von wenigstens einem Anti-Krebs-Arzneistoff sowie verschiedenen Tumormarkern umfasst.

## Revendications

1. Dispositif microfluidique pour conduire des dosages immunologiques multiplexes pour détecter plusieurs analytes cibles dans un échantillon liquide en utilisant un ensemble d'étiquettes et d'anti-étiquettes, le dispositif comprenant un réseau d'anti-étiquettes différentes immobilisées sur une surface d'un capteur pour mesurer un signal, chaque anti-étiquette étant agencée pour immobilier un anticorps étiqueté ou un antigène étiqueté, séparant ainsi lors d'une utilisation des complexes immuns étiquetés de la phase liquide affectant le signal mesuré ;
le dispositif comprenant un compartiment capteur pour que des complexes immuns étiquetés se forment dans la phase liquide, et le dispositif comprenant une ou plusieurs zones de détection comprenant le réseau d'anti-étiquettes différentes pour l'immobilisation de complexes immuns étiquetés ;
le dispositif comprenant un canal de retard microfluidique entre le compartiment capteur et la ou les zones de détection pour augmenter le temps d'incubation de réactifs en phase liquide qui réagissent pour former des complexes immuns étiquetés avant d'atteindre les anti-étiquettes ; et, lors d'une utilisation, du liquide comprenant les complexes immuns étiquetés s'écoulant ou étant entraîné de manière fluidique du compartiment capteur à la ou les zone de détection.

2. Dispositif selon la revendication 1, la mesure de détection/signal comprenant une détection colorimétrique, une détection fluorimétrique, une spectroscopie d'impédance faradique ou non faradique ou une détection de précipité actif rédox.

3. Dispositif selon la revendication 1 ou la revendication 2, soit i) une partie de la surface du capteur comprenant un polymère conducteur ou semiconducteur, soit ii) la surface du capteur comprenant la surface d'une ou plusieurs électrodes.

4. Dispositif selon une quelconque revendication précédente, le dispositif comprenant un réseau d'électrodes comportant une anti-étiquette différente sur chaque électrode, éventuellement, chaque entitéétiquette étant adsorbée sur ou liée chimiquement à l'électrode, et éventuellement l'électrode étant une électrode d'or.

5. Dispositif selon une quelconque revendication précédente, les analytes cibles comprenant des anticorps et/ou des antigènes.

6. Dispositif selon une quelconque revendication précédente, chaque anti-étiquette étant un anticorps, un aptamère, un affimère ou un polymère imprimé de manière moléculaire (MIP).

7. Dispositif selon la revendication 6, dans lequel lorsque une anti-étiquette est un anticorps, l'anticorps anti-étiquette est avidine/anti-biotine et l'étiquette correspondante est la biotine ou un anticorps étiquette anti-flag^{®} contre une étiquette flag^{®}, ou un anticorps anti-His-6 contre His-6, ou un anticorps anti-c-myc contre c-myc, ou un anticorps de peptide accepteur d'anti-biotine contre un peptide accepteur de biotine, ou un anticorps anti-V5 contre V5 tag ou un anticorps anti-glutathion S-transférase (GST) contre une étiquette glutathion S-transférase, ou l'anticorps anti-étiquette est ab anti-souris de mouton et l'étiquette correspondante est ab primaire, p. ex., souris, ou anti-FITC contre FITC, ou anti-HRP contre HRP, ou anti-MPB contre la protéine de liaison au maltose R; ou antistreptavidine/biotine contre la streptavidine.

8. Dispositif selon une quelconque revendication précédente, le dispositif comprenant un réseau d'au moins : 3 ou 4 ou 5 ou 6 ou 7 ou 8 ou 9 ou 10 anti-étiquettes différentes.

9. Dispositif selon une quelconque revendication précédente, le dispositif comprenant un réseau de 6 anti-étiquettes différentes, éventuellement les 6 anti-étiquettes différentes étant des anticorps contre : FITC ou biotin, Flag, His-6, c-Myc, V5 et GST.

10. Dispositif selon une quelconque revendication précédente, pour un test d'une maladie infectieuse ou un test cardiaque ou un test de médicaments ou un test de statut immunologique/allergie.

11. Dispositif selon une quelconque revendication précédente, le dosage multiplexe comprenant un ou plusieurs des formats de dosage immunologique suivants :
a. dosage immunométrique ou en sandwich à deux sites avec séparation d'anti-étiquette
b. dosage immunologique compétitif avec séparation d'anti-étiquette
c. dosage immunologique compétitif (inversé) avec séparation d'anti-étiquette
d. dosage immunologique direct pour la détection d'anticorps avec séparation d'anti-étiquette
e. dosage immunologique indirect pour la détection d'anticorps avec séparation d'anti-étiquette.

12. Dispositif selon une quelconque revendication précédente, le réseau d'anti-étiquettes différentes étant agencé pour conduire des dosages immunologiques d'au moins deux formats différents sur un seul dispositif.

13. Dispositif selon la revendication 12, le réseau d'anti-étiquettes étant agencé pour détecter de faibles taux de cortisol tout en quantifiant également des taux plus élevés d'immunoglobuline G et d'immunoglobuline A, et éventuellement également en détectant la testostérone et/ou l'immunoglobuline M ; le dispositif comprenant un format de dosage immunologique compétitif pour détecter le cortisol et éventuellement la testostérone, et un format de réseau en sandwich pour détecter les immunoglobulines G et A et éventuellement l'immunoglobuline M.

14. Dispositif selon la revendication 12, le dispositif comprenant un panel d'anti-étiquettes pour la détection d'au moins un médicament anticancéreux ainsi que divers marqueurs tumoraux.
